# EUROPEAN PATENT APPLICATION

(11) **EP 3 872 092 A1**
(43) Date of publication of application: **01.09.2021**
(21) Application number: 19872158.1
(22) Date of filing: 27.09.2019
(51) Int. Cl.: C07K 16/18, C12N 5/20, G01N 33/577

(54) **RECOMBINANT ANTIBODY OF ANTI-HUMAN CARDIAC TROPONIN I**

(30) Priority: 10.10.2018 CN 201811180362
(71) Applicant: Fapon Biotech Inc., Shenzhen City , Guangdong 518063 (CN)
(72) Inventor: CUI, Peng, Dongguan, Guangdong 523000 (CN); HE, Zhiqiang, Dongguan, Guangdong 523000 (CN); MENG, Yuan, Dongguan, Guangdong 523000 (CN); HAN, Ricai, Dongguan, Guangdong 523000 (CN); ZHONG, Dongmei, Dongguan, Guangdong 523000 (CN); FAN, Lingyun, Dongguan, Guangdong 523000 (CN)
(74) Representative: Gill, Siân Victoria
(86) International application number: PCT/CN2019/108681
(87) International publication number: WO 2020/073831

(57) **Abstract**

Provided are an isolated binding protein including an antigen binding domain of cardiac troponin I (cTnI), as well as a preparation and uses of the binding protein. The antigen binding domain includes at least one complementary determining region selected from the amino acid sequences defined herein; or the antigen-binding domain has at least 80% sequence identity with the complementary determining regions of said amino acid sequences and has an affinity for cardiac troponin I of K_{D} ≤ 3.33×10⁻⁸ mo/L. The binding protein may be used in the field of cTnI protein detection.

## Description

### CROSS-REFERENCE TO RELATED APPLICATIONS

The present application claims a priority to Chinese Patent Application No. 201811180362.8, titled "Recombinant Antibody of Anti-human Cardiac Troponin", filed on October 10, 2018 in the China Patent Office, which is hereby incorporated by reference in its entirety.

### TECHNICAL FIELD

The present disclosure relates to the technical field of immunology, and particularly, to a recombinant antibody against human cardiac troponin I.

### BACKGROUND

Before the 1980s, the World Health Organization (WHO) has always used myocardial enzyme activity as one of the diagnostic criteria for acute myocardial infarction (AMI). In the late 1980s, researchers discovered that troponin (Tn) is more sensitive and specific than creatine phosphokinase (CK), creatine phosphokinase isoenzyme (CK-MB), lactate dehydrogenase, aspartate aminotransferase, and other biomarkers. Cardiac troponin I (cTnI) exists only in the myocardium and is a marker of myocardial cells, whose abnormal changes can affect the systolic and diastolic functions of the heart and thus can be used to diagnose myocardial necrosis, determine myocardial injury, etc. The cardiac troponin I becomes one of the most sensitive and specific markers of myocardial cell injury, and is recognized as the main biochemical marker for rapidly diagnosing AMI and acute coronary syndromes (ACS), as well as assisting ACS risk stratification and reflecting its prognosis.

A content of cTnI in normal people's blood is generally less than 0.3 µg/L. When the integrity of the myocardial cell membrane is damaged by ischemia or hypoxia, free cTnI can quickly penetrate the cell membrane and enter the bloodstream. Therefore, a rapid, sensitive and accurate determination of cTnI in human blood and a changing trend thereof at an early stage of onset is clinically significant for a diagnosis of acute myocardial infarction, a risk stratification of acute coronary syndromes, and monitoring of myocardial injury caused by various factors. Clinical methods for detecting cTnI levels include enzyme-linked immunosorbent assay (ELISA), chemiluminescence, colloidal gold, etc. Different methods have their own advantages and disadvantages, but they all require specific monoclonal antibodies directed against cTnI.

Existing cTnI antibodies, due to their low activity and poor affinity, cannot be well applied to the detection of cTnI protein. Therefore, there is a strong demand in the art for antibodies that effectively and specifically bind to and detect cTnI.

### SUMMARY

The present disclosure relates to a novel isolated binding protein containing cardiac troponin I (cTnI) antigen-binding domain, and studies on a preparation and use of the binding protein.

The antigen-binding domain includes at least one complementary determining region selected from the following amino acid sequences; or the antigen-binding domain has at least 80% sequence identity with the complementary determining regions of the following amino acid sequences and has an affinity for cardiac troponin I of K_{D} ≤ 3.33×10⁻⁸ mo/L:
a complementary determining region CDR-VH1 of G-F-T-X1-S-N-Y-A-M-S, where X1 is Y or F;
a complementary determining region CDR-VH2 of I-S-X1-G-G-S-Y-S-X2-Y-P-D-S-V-K, where X1 is T or S, and X2 is Y or F;
a complementary determining region CDR-VH3 of A-R-X1-D-N-Y-X2-G-S-X3-F-M-D-Y, where X1 is K or R, X2 is Y, P or F, and X3 is T or S;
a complementary determining region CDR-VL1 of R-A-S-Q-X1-I-T-X2-Y-L-N, where X1 is D or E, and X2 is Q or N;
a complementary determining region CDR-VL2 of I-Y-Y-X1-S-R-X2-H-S-G-V-S, where X1 is S or T, and X2 is I or L; and
a complementary determining region CDR-VL3 of Q-Q-G-X1-T-X2-P-L-T, where X1 is N or H, and X2 is I or L.

An important advantage is in that the binding protein has strong activity and has a high affinity for human cTnI protein.

In one or more embodiments:
in the complementary determining region CDR-VH2, X1 is S;
in the complementary determining region CDR-VH3, X1 is R;
in the complementary determining region CDR-VL1, X2 is N; and
in the complementary determining region CDR-VL2, X1 is T.

In one or more embodiments, in the complementary determining region CDR-VH1, X1 is Y.

In one or more embodiments, in the complementary determining region CDR-VH1, X1 is F.

In one or more embodiments, in the complementary determining region CDR-VH2, X2 is Y.

In one or more embodiments, in the complementary determining region CDR-VH2, X2 is F.

In one or more embodiments, in the complementary determining region CDR-VH3, X2 is Y, and X3 is T.

In one or more embodiments, in the complementary determining region CDR-VH3, X2 is P, and X3 is T.

In one or more embodiments, in the complementary determining region CDR-VH3, X2 is F, and X3 is T.

In one or more embodiments, in the complementary determining region CDR-VH3, X2 is Y, and X3 is S.

In one or more embodiments, in the complementary determining region CDR-VH3, X2 is P, and X3 is S.

In one or more embodiments, in the complementary determining region CDR-VH3, X2 is F, and X3 is S.

In one or more embodiments, in the complementary determining region CDR-VL1, X1 is D.

In one or more embodiments, in the complementary determining region CDR-VL1, X1 is E.

In one or more embodiments, in the complementary determining region CDR-VL2, X2 is I.

In one or more embodiments, in the complementary determining region CDR-VL2, X2 is L.

In one or more embodiments, in the complementary determining region CDR-VL3, X1 is N, and X2 is I.

In one or more embodiments, in the complementary determining region CDR-VL3, X1 is H, and X2 is I.

In one or more embodiments, in the complementary determining region CDR-VL3, X1 is N, and X2 is L.

In one or more embodiments, in the complementary determining region CDR-VL3, X1 is H, and X2 is L.

In one or more embodiments, a mutation site of each of the complementary determining regions is selected from any one of the following mutation combinations:

| Site | CDR-VH1 X1 | CDR-VH2 X2 | CDR-VH3 X2/X3 | CDR-VL1 X1 | CDR-VL2 X2 | CDR-VL3 X1/X2 |
|---|---|---|---|---|---|---|
| Mutation combination 1 | Y | Y | Y/T | D | I | N/I |
| Mutation combination 2 | F | F | Y/S | E | L | H/L |
| Mutation combination 3 | Y | F | P/T | E | I | N/I |
| Mutation combination 4 | F | Y | P/S | D | L | H/L |
| Mutation combination 5 | Y | Y | F/T | E | L | N/L |
| Mutation combination 6 | F | F | F/S | E | I | H/L |
| Mutation combination 7 | Y | F | Y/S | D | L | N/I |
| Mutation combination 8 | F | Y | F/T | E | I | H/I |
| Mutation combination 9 | Y | Y | F/S | E | I | H/I |
| Mutation combination 10 | F | F | Y/T | D | L | N/L |
| Mutation combination 11 | Y | F | Y/S | D | I | H/L |
| Mutation combination 12 | F | Y | Y/T | D | L | N/I |
| Mutation combination 13 | Y | Y | P/T | E | L | H/L |
| Mutation combination 14 | F | F | P/S | E | I | N/I |
| Mutation combination 15 | Y | F | F/T | D | L | H/I |
| Mutation combination 16 | F | Y | F/S | E | I | N/L |
| Mutation combination 17 | Y | Y | Y/T | D | I | N/I |
| Mutation combination 18 | F | F | Y/S | D | L | H/I |
| Mutation combination 19 | Y | F | P/S | E | I | N/L |
| Mutation combination 20 | F | Y | F/T | E | L | H/L |
| Mutation combination 21 | Y | Y | F/S | D | L | N/L |
| Mutation combination 22 | F | F | Y/T | D | I | H/L |
| Mutation combination 23 | Y | F | Y/S | D | I | H/I |
| Mutation combination 24 | F | Y | P/T | E | I | H/I |
| Mutation combination 25 | Y | Y | F/T | E | I | H/I |
| Mutation combination 26 | F | F | F/S | D | L | N/L |
| Mutation combination 27 | Y | F | Y/T | E | I | H/L |
| Mutation combination 28 | F | Y | Y/S | D | L | N/I |
| Mutation combination 29 | Y | Y | P/T | D | L | H/L |
| Mutation combination 30 | F | F | F/S | E | I | N/I |
| Mutation combination 31 | Y | F | F/S | E | L | H/I |
| Mutation combination 32 | F | Y | Y/T | D | I | N/L |
| Mutation combination 33 | Y | Y | Y/S | D | I | N/I |
| Mutation combination 34 | F | F | P/T | D | L | H/I |
| Mutation combination 35 | Y | F | P/S | D | I | N/I |
| Mutation combination 36 | F | Y | F/T | D | L | H/L |
| Mutation combination 37 | Y | Y | Y/T | E | I | N/L |
| Mutation combination 38 | F | F | Y/S | D | L | H/L |
| Mutation combination 39 | Y | F | P/T | D | L | N/I |
| Mutation combination 40 | F | Y | P/S | D | I | H/I |
| Mutation combination 41 | Y | Y | F/T | E | I | N/L |
| Mutation combination 42 | F | F | F/S | E | L | N/L |
| Mutation combination 43 | Y | F | Y/S | E | I | H/I |
| Mutation combination 44 | F | Y | P/T | D | L | N/I |
| Mutation combination 45 | Y | Y | P/S | D | L | H/L |
| Mutation combination 46 | F | F | F/T | E | I | N/I |
| Mutation combination 47 | Y | F | Y/T | E | L | H/L |
| Mutation combination 48 | F | Y | P/S | D | I | N/L |
| Mutation combination 49 | Y | Y | P/T | E | I | N/I |

In one or more embodiments, the binding protein includes at least 3 CDRs (for example, 3 heavy chain CDRs or 3 light chain CDRs); or the binding protein includes at least 6 CDRs.

In one or more embodiments, the binding protein is a complete antibody including variable and constant regions.

In one or more embodiments, the binding protein is a "functional fragment" of an antibody, such as one of nanobody, F(ab')2, Fab', Fab, Fv, scFv, a bispecific antibody, and a minimum recognition unit of an antibody.

In one or more embodiments, the binding protein includes light chain framework regions FR-L1, FR-L2, FR-L3, and FR-L4 that have sequences set forth as SEQ ID NO: 1 to SEQ ID NO: 4, respectively, and/or heavy chain framework regions FR-H1, FR-H2, FR-H3 and FR-H4 that have sequences set forth as SEQ ID NO: 5 to SEQ ID NO: 8, respectively.

In one or more embodiments, the binding protein further includes an antibody constant region sequence.

In one or more embodiments, the constant region sequence is a sequence of constant region selected from any one of IgG1, IgG2, IgG3, IgG4, IgA, IgM, IgE, and IgD.

In one or more embodiments, the constant region is derived from a species of cattle, horse, dairy cow, pig, sheep, goat, rat, mouse, dog, cat, rabbit, camel, donkey, deer, marten, chicken, duck, goose, turkey, cockfight, or human.

In one or more embodiments, the constant region is derived from a mouse;
a light chain constant region sequence is set forth as SEQ ID NO: 9; and
a heavy chain constant region sequence is set forth as SEQ ID NO: 10.

The present disclosure further relates to an isolated nucleic acid molecule. The nucleic acid molecule is DNA or RNA that encodes the binding protein as described above.

The present disclosure further relates to a vector, including the nucleic acid molecule as described above.

The present disclosure further relates to a host cell, transformed with the vector as described above.

The host cell may be a eukaryotic cell, such as a mammalian cell.

The present disclosure further relates to a method for producing the binding protein as described above. The method includes the following steps: culturing the host cell as described above in a medium under suitable culture conditions, and collecting the produced binding protein from the medium or from the cultured host cell.

The present disclosure further relates to a use of the binding protein as described above in a preparation of a diagnostic agent or kit for diagnosing acute myocardial infarction, acute coronary syndrome, pulmonary infarction, unstable angina, or myocardial injury.

The present disclosure further relates to a method for detecting a troponin I antigen in a test sample. The method includes:
a) under conditions allowing an antibody/antigen binding reaction to occur, contacting the troponin I antigen in the test sample with the binding protein as described above to form an immune complex; and
b) detecting a presence of the immune complex, the presence of the immune complex indicating a presence of the troponin I antigen in the test sample.

In this embodiment, the binding protein can be labeled with an indicator for displaying signal intensity, allowing the complex to be detected easily.

In one or more embodiments, in step a), the immune complex further includes a second antibody, and the second antibody binds to the binding protein.

In one or more embodiments, in step a), the immune complex further includes a second antibody, and the second antibody binds to the troponin I antigen.

In an aspect of the present disclosure, the present disclosure further relates to a kit including the binding protein as described above.

In one or more embodiments, the troponin I antigen is cardiac troponin I antigen.

The present disclosure further relates to a use of the binding protein as described above in a diagnosis of a cardiac troponin I-related disease.

The present disclosure further relates to a method for diagnosing a cardiac troponin I-related disease. The method includes:
A) under conditions allowing a binding reaction to occur, contacting a sample from a subject with the binding protein according to the present disclosure, to perform the binding reaction; and
B) detecting an immune complex produced in the binding reaction,
   in which a presence of the immune complex indicates a presence of the cardiac troponin I-related disease.

In one or more embodiments, the method is based on fluorescence immunoassay, chemiluminescence, colloidal gold immunoassay, radioimmunoassay, and/or enzyme-linked immunoassay.

In one or more embodiments, the sample is selected from at least one of whole blood, peripheral blood, serum, plasma, or myocardial tissue.

In one or more embodiments, the subject is a mammal, preferably a primate, and more preferably a human.

In one or more embodiments, the cardiac troponin I-related disease is a cardiovascular disease.

In one or more embodiments, the cardiac troponin I-related disease is selected from the group consisting of acute myocardial infarction, acute coronary syndrome, pulmonary infarction, unstable angina, myocardial injury, and combinations thereof.

### DESCRIPTION OF EMBODIMENTS

The present disclosure can be more easily understood through the following description of some embodiments of the present disclosure and the details of examples included therein.

Before further describing the present disclosure, it should be understood that the present disclosure is not limited to specific embodiments described below, as these embodiments are necessarily diverse. It should also be understood that the terms used in the specification are merely for illustrative purposes, instead of serving as limitations, as the scope of the present disclosure shall be defined only in the pending claims.

### Definition of terms

The term "isolated binding protein including an antigen-binding domain" refers broadly to all proteins/protein fragments that include a CDR region. The term "antibody" includes polyclonal and monoclonal antibodies and antigen-compound-binding fragments of these antibodies, including Fab, F(ab')2, Fd, Fv, scFv, bispecific antibodies, and the minimum recognition units of antibodies, as well as single-chain derivatives of these antibodies and fragments. The type of antibody can be selected from IgG1, IgG2, IgG3, IgG4, IgA, IgM, IgE or IgD. In addition, the term "antibody" used herein includes natural antibodies and non-natural antibodies, including, for example, chimeric, bifunctional, and humanized antibodies, and related synthetic isoforms. The term "antibody" used herein is interchangeable with "immunoglobulin".

The "variable region" or "variable domain" of an antibody refers to an amino-terminal domain of a heavy or light chain of an antibody. The variable domain of a heavy chain can be referred to as "VH". The variable domain of a light chain can be referred to as "VL". These domains are usually the most variable part of an antibody and contain an antigen binding site. The light or heavy chain variable region (VL or VH) is composed of three hypervariable regions called "complementary determining regions" or "CDRs", and framework regions separating the three complementary determining regions. Ranges of the framework region and the CDR have been precisely defined, for example, in Kabat (see "Sequences of Proteins of Immunological Interests", E. Kabat, et al., US Department of Health and Human Services, 1983) and Chothia. The framework region of an antibody serves to locate and align CDRs, and the CDRs are mainly responsible for binding to antigens.

As used herein, "backbone region", "framework region", or "FR" means a region of an antibody variable domain other than those defined as CDRs. The frameworks of each antibody variable domain can be further subdivided into adjacent regions FR1, FR2, FR3, and FR4 that are separated by CDRs.

In general, the light or heavy chain variable region VL/VH can be obtained by connecting the numbered CDRs and FRs in the following combinations: FR1-CDR1-FR2-CDR2-FR3-CDR3- FR4.

As used herein, the term "purified" or "isolated" in association with a polypeptide or nucleic acid means that the polypeptide or nucleic acid is not in its natural medium or in its natural form. Accordingly, the term "isolated" includes: extracting polypeptides or nucleic acids, for example, if they are naturally occurring, from natural environment or natural form. For example, the isolated polypeptides generally do not contain at least some proteins or other cellular components that are usually bound to it or mixed with it or exist in solution. The isolated polypeptides include the naturally occurring polypeptides contained in cell lysates, the polypeptides in purified or partially purified form, recombinant polypeptides, the polypeptides expressed or secreted by cells, and the polypeptides in heterologous host cells or cultures. In connection with nucleic acids, the term "isolated" or "purified" indicates that the nucleic acid is not in its natural genomic background, for example, in a vector, as an expression cassette, linked to a promoter, or artificially introduced into heterologous host cells.

As used herein, the term "bispecific antibody" or "bifunctional antibody" refers to an artificial hybrid binding protein having two different pairs of heavy/light chains and two different binding sites. The bispecific binding protein can be produced by a variety of methods, including fusion hybridomas or linking of Fab' fragments.

As used herein, the term "sequence identity" refers to the similarity between at least two different sequences. The identity percentage can be determined by standard algorithms, such as Basic Local Alignment Search Tool (BLAST); Needleman's algorithm, etc.; or Meyers's algorithm, etc. In one or more embodiments, a set of parameters may be a blocks substitution matrix (Blosum 62), and a gap penalty of 12, a gap extension penalty of 4, and a frameshift gap penalty of 5. In one or more embodiments, the identity percentage between two amino acid or nucleotide sequences can also be determined using the algorithm by Meyers and Miller ((1989) CABIOS 4: 11-17), which has been incorporated into ALIGN program (version 2.0), using a PAM120 weighted residue table, gap length penalty of 12, and gap penalty of 4. The identity percentage is usually calculated by comparing the similar length of the sequences.

As used herein, the term "affinity" refers to a binding strength of an antigen-binding domain of a binding protein or antibody to an antigen or epitope. The affinity can be measured by KD value, the smaller the KD value, the greater the affinity.

### Exemplary embodiments of the present disclosure

The present disclosure provides an isolated binding protein including an antigen-binding domain. The antigen-binding domain includes at least one complementary determining region selected from the following amino acid sequences; or the antigen-binding domain has at least 80% sequence identity with the complementary determining regions of the following amino acid sequences and has an affinity for cardiac troponin I of K_{D} ≤ 3.33×10⁻⁸ mol/L:
a complementary determining region CDR-VH1 of A-S-G-F-X1-F-X2-T-F-A-M-S, where
a complementary determining region CDR-VH1 of G-F-T-X1-S-N-Y-A-M-S, where X1 is Y or F;
a complementary determining region CDR-VH2 of I-S-X1-G-G-S-Y-S-X2-Y-P-D-S-V-K, where X1 is T or S, and X2 is Y or F;
a complementary determining region CDR-VH3 of A-R-X1-D-N-Y-X2-G-S-X3-F-M-D-Y, where X1 is K or R, X2 is Y, P or F, and X3 is T or S;
a complementary determining region CDR-VL1 of R-A-S-Q-X1-I-T-X2-Y-L-N, where X1 is D or E, and X2 is Q or N;
a complementary determining region CDR-VL2 of I-Y-Y-X1-S-R-X2-H-S-G-V-S, where X1 is S or T, and X2 is I or L; and
a complementary determining region CDR-VL3 of Q-Q-G-X1-T-X2-P-L-T, where X1 is N or H, and X2 is I or L.

In one or more embodiments, the antigen-binding domain has at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity with the complementary determining regions of the following amino acid sequences; and the antigen-binding domain has an affinity for the cardiac troponin I of KD ≤3.33×10⁻⁸ mol/L, for example, 1×10⁻⁹ mol/L, 2×10⁻⁹ mol/L, 3×10⁻⁹ mol/L, 4×10⁻⁹ mol/L, 4.5×10⁻⁹ mol/L, 5×10⁻⁹ mol/L, 6×10⁻⁹ mol/L, 7×10⁻⁹ mol/L, 8×10⁻⁹ mol/L, 9×10⁻⁹ mol/L, 1×10⁻¹⁰ mol/L, 3×10⁻¹⁰ mol/L, 5×10⁻¹⁰ mol/L, 7×10⁻¹⁰ mol/L, or 9×10⁻¹⁰ mol/L; or 8.30×10⁻¹⁰ mol/L ≤ K_{D} < 3.33×10⁻⁸ mol/L, or KD smaller than or equal to 1×10⁻⁹ mol/L, 2×10⁻⁹ mol/L, 3×10⁻⁹ mol/L, 4×10⁻⁹ mol/L, 4.5×10⁻⁹ mol/L, 5×10⁻⁹ mol/L, 6×10⁻⁹ mol/L, 7×10⁻⁹ mol/L, 8×10⁻⁹ mol/L, 9×10⁻⁹ mol/L, 1×10⁻¹⁰ mol/L, 3×10⁻¹⁰ mol/L, 5×10⁻¹⁰ mol/L, 7×10⁻¹⁰ mol/L, or 9×10⁻¹⁰ mol/L.

The affinity is measured with the method as described in the present disclosure.

In one or more embodiments:
in the complementary determining region CDR-VH2, X1 is S;
in the complementary determining region CDR-VH3, X1 is R;
in the complementary determining region CDR-VL1, X2 is N; and
in the complementary determining region CDR-VL2, X1 is T.

In one or more embodiments, in the complementary determining region CDR-VH1, X1 is Y.

In one or more embodiments, in the complementary determining region CDR-VH1, X1 is F.

In one or more embodiments, in the complementary determining region CDR-VH2, X2 is Y.

In one or more embodiments, in the complementary determining region CDR-VH2, X2 is F.

In one or more embodiments, in the complementary determining region CDR-VH3, X2 is Y, and X3 is T.

In one or more embodiments, in the complementary determining region CDR-VH3, X2 is P, and X3 is T.

In one or more embodiments, in the complementary determining region CDR-VH3, X2 is F, and X3 is T.

In one or more embodiments, in the complementary determining region CDR-VH3, X2 is Y, and X3 is S.

In one or more embodiments, in the complementary determining region CDR-VH3, X2 is P, and X3 is S.

In one or more embodiments, in the complementary determining region CDR-VH3, X2 is F, and X3 is S.

In one or more embodiments, in the complementary determining region CDR-VL1, X1 is D.

In one or more embodiments, in the complementary determining region CDR-VL1, X1 is E.

In one or more embodiments, in the complementary determining region CDR-VL2, X2 is I.

In one or more embodiments, in the complementary determining region CDR-VL2, X2 is L.

In one or more embodiments, in the complementary determining region CDR-VL3, X1 is N, and X2 is I.

In one or more embodiments, in the complementary determining region CDR-VL3, X1 is H, and X2 is I.

In one or more embodiments, in the complementary determining region CDR-VL3, X1 is N, and X2 is L.

In one or more embodiments, in the complementary determining region CDR-VL3, X1 is H, and X2 is L.

In one or more embodiments, a mutation site of each of the complementary determining regions is selected from any one of the following mutation combinations:

| Site | CDR-VH1 X1 | CDR-VH2 X2 | CDR-VH3 X2/X3 | CDR-VL1 X1 | CDR-VL2 X2 | CDR-VL3 X1/X2 |
|---|---|---|---|---|---|---|
| Mutation combination 1 | Y | Y | Y/T | D | I | N/I |
| Mutation combination 2 | F | F | Y/S | E | L | H/L |
| Mutation combination 3 | Y | F | P/T | E | I | N/I |
| Mutation combination 4 | F | Y | P/S | D | L | H/L |
| Mutation combination 5 | Y | Y | F/T | E | L | N/L |
| Mutation combination 6 | F | F | F/S | E | I | H/L |
| Mutation combination 7 | Y | F | Y/S | D | L | N/I |
| Mutation combination 8 | F | Y | F/T | E | I | H/I |
| Mutation combination 9 | Y | Y | F/S | E | I | H/I |
| Mutation combination 10 | F | F | Y/T | D | L | N/L |
| Mutation combination 11 | Y | F | Y/S | D | I | H/L |
| Mutation combination 12 | F | Y | Y/T | D | L | N/I |
| Mutation combination 13 | Y | Y | P/T | E | L | H/L |
| Mutation combination 14 | F | F | P/S | E | I | N/I |
| Mutation combination 15 | Y | F | F/T | D | L | H/I |
| Mutation combination 16 | F | Y | F/S | E | I | N/L |
| Mutation combination 17 | Y | Y | Y/T | D | I | N/I |
| Mutation combination 18 | F | F | Y/S | D | L | H/I |
| Mutation combination 19 | Y | F | P/S | E | I | N/L |
| Mutation combination 20 | F | Y | F/T | E | L | H/L |
| Mutation combination 21 | Y | Y | F/S | D | L | N/L |
| Mutation combination 22 | F | F | Y/T | D | I | H/L |
| Mutation combination 23 | Y | F | Y/S | D | I | H/I |
| Mutation combination 24 | F | Y | P/T | E | I | H/I |
| Mutation combination 25 | Y | Y | F/T | E | I | H/I |
| Mutation combination 26 | F | F | F/S | D | L | N/L |
| Mutation combination 27 | Y | F | Y/T | E | I | H/L |
| Mutation combination 28 | F | Y | Y/S | D | L | N/I |
| Mutation combination 29 | Y | Y | P/T | D | L | H/L |
| Mutation combination 30 | F | F | F/S | E | I | N/I |
| Mutation combination 31 | Y | F | F/S | E | L | H/I |
| Mutation combination 32 | F | Y | Y/T | D | I | N/L |
| Mutation combination 33 | Y | Y | Y/S | D | I | N/I |
| Mutation combination 34 | F | F | P/T | D | L | H/I |
| Mutation combination 35 | Y | F | P/S | D | I | N/I |
| Mutation combination 36 | F | Y | F/T | D | L | H/L |
| Mutation combination 37 | Y | Y | Y/T | E | I | N/L |
| Mutation combination 38 | F | F | Y/S | D | L | H/L |
| Mutation combination 39 | Y | F | P/T | D | L | N/I |
| Mutation combination 40 | F | Y | P/S | D | I | H/I |
| Mutation combination 41 | Y | Y | F/T | E | I | N/L |
| Mutation combination 42 | F | F | F/S | E | L | N/L |
| Mutation combination 43 | Y | F | Y/S | E | I | H/I |
| Mutation combination 44 | F | Y | P/T | D | L | N/I |
| Mutation combination 45 | Y | Y | P/S | D | L | H/L |
| Mutation combination 46 | F | F | F/T | E | I | N/I |
| Mutation combination 47 | Y | F | Y/T | E | L | H/L |
| Mutation combination 48 | F | Y | P/S | D | I | N/L |
| Mutation combination 49 | Y | Y | P/T | E | I | N/I |

In one or more embodiments, X1 in the six CDRs of the binding protein described in the present disclosure each independently represents the amino acid defined in the present disclosure; X2 in the six CDRs of the binding protein described in the present disclosure each independently represents the amino acids defined in the present disclosure; and X3 in the six CDRs of the binding protein described in the present disclosure each independently represents the amino acids defined in the present disclosure.

In one or more embodiments, the binding protein includes at least 3 CDRs (for example, 3 heavy chain CDRs or 3 light chain CDRs); or the binding protein includes at least 6 CDRs.

In one or more embodiments, the binding protein is a complete antibody including variable and constant regions.

In one or more embodiments, the binding protein is a "functional fragment" of an antibody, such as one of nanobody, F(ab')2, Fab', Fab, Fv, scFv, a bispecific antibody, and a minimum recognition unit of an antibody.
scFv (sc = single chain), bispecific antibody (diabodies).

The "functional fragment" described in the present disclosure particularly refers to an antibody fragment having the same specificity to cTnI as a maternal antibody. In addition to the above functional fragments, the "functional fragment" further includes any fragments whose half-life has been increased.

These functional fragments usually have the same binding specificity as the antibodies from which they are derived. In view of the present disclosure, those skilled in the art are able to infer that the antibody fragments of the present disclosure can obtain the above-mentioned functional fragments through methods such as enzymatic digestion (including pepsin or papain) and/or methods of splitting disulfide bonds by chemical reduction.

The antibody fragments can also be obtained by peptide synthesis with recombinant genetic techniques well-known to those skilled in the art or with, for example, automatic peptide synthesizers such as those purchased from Applied BioSystems.

In one or more embodiments, the binding protein includes light chain framework regions FR-L1, FR-L2, FR-L3, and FR-L4 that have sequences set forth as SEQ ID NO: 1 to SEQ ID NO: 4, respectively, and/or heavy chain framework regions FR-H1, FR-H2, FR-H3 and FR-H4 that have sequences set forth as SEQ ID NO: 5 to SEQ ID NO: 8, respectively.

It should be understood that, in addition to the amino acid sequences disclosed above, the species source of the framework regions may also be human, so as to constitute a humanized antibody.

In one or more embodiments, the binding protein further includes an antibody constant region sequence.

In one or more embodiments, the constant region sequence is a sequence of constant region selected from any one of IgG1, IgG2, IgG3, IgG4, IgA, IgM, IgE, and IgD.

In one or more embodiments, the constant region is derived from a species of cattle, horse, dairy cow, pig, sheep, goat, rat, mouse, dog, cat, rabbit, camel, donkey, deer, marten, chicken, duck, goose, turkey, cockfight, or human.

In one or more embodiments, the constant region is derived from a mouse;
a light chain constant region sequence is set forth as SEQ ID NO: 9; and
a heavy chain constant region sequence is set forth as SEQ ID NO: 10.

The present disclosure further relates to an isolated nucleic acid molecule. The nucleic acid molecule is DNA or RNA that encodes the binding protein as described above.

The present disclosure further relates to a vector, including the nucleic acid molecule as described above.

The present disclosure further includes at least one nuclear construct encoding the nucleic acid molecule as described above, for example, a plasmid such as an expression plasmid, and in an embodiment of the present disclosure, a method for constructing the vector will be described.

The present disclosure further relates to a host cell, transformed with the vector as described above.

The host cell may be a eukaryotic cell, such as a mammalian cell.

In one or more embodiments, the host cell is a CHO cell.

The present disclosure further relates to a method for producing the binding protein as described above. The method includes the following steps: culturing the host cell as described above in a medium under suitable culture conditions, and collecting the produced binding protein from the medium or from the cultured host cell.

The present disclosure further relates to a use of the binding protein as described above in a preparation of a diagnostic agent or kit for diagnosing acute myocardial infarction, acute coronary syndrome, pulmonary infarction, unstable angina, or myocardial injury.

The present disclosure further relates to a method for detecting a troponin I antigen in a test sample. The method includes:
a) under conditions allowing an antibody/antigen binding reaction to occur, contacting the troponin I antigen in the test sample with the binding protein as described above to form an immune complex; and
b) detecting a presence of the immune complex, the presence of the immune complex indicating a presence of the troponin I antigen in the test sample.

In this embodiment, the binding protein can be labeled with an indicator for displaying signal intensity, allowing the complex to be detected easily.

In one or more embodiments, in step a), the immune complex further includes a second antibody, and the second antibody binds to the binding protein.

In this embodiment, the binding protein, in a form of a first antibody, and the second antibody form paired antibodies for binding to different epitopes of cTnI.

The second antibody can be labeled with an indicator for displaying signal intensity, allowing the complex to be detected easily.

In one or more embodiments, in step a), the immune complex further includes a second antibody, and the second antibody binds to the troponin I antigen.

In this embodiment, the binding protein serves as an antigen of the second antibody, and the second antibody can be labeled with an indicator for displaying signal intensity, allowing the complex to be detected easily.

In one or more embodiments, the indicator for displaying signal strength includes any one of fluorescent substance, quantum dot, digoxigenin-labeled probe, biotin, radioisotope, radioactive contrast agent, paramagnetic ion fluorescent microsphere, electron dense substance, chemiluminescent label, ultrasound contrast agent, photosensitizer, colloidal gold, or enzyme.

In one or more embodiments, the fluorescent substance includes any one of: Alexa 350, Alexa 405, Alexa 430, Alexa 488, Alexa 555, Alexa 647, AMCA, aminoacridine, BODIPY 630/650, BODIPY 650/665, BODIPY-FL, BODIPY-R6G, BODIPY-TMR, BODIPY-TRX, 5-carboxy-4',5'-dichloro-2',7'-dimethoxyfluorescein, 5-carboxy-2' ,4',5',7'-tetrachlorofluorescein, 5-carboxyfluorescein, 5-carboxyrhodamine, 6-carboxyrhodamine, 6-carboxytetramethylrhodamine, Cascade Blue, Cy2, Cy3, Cy5, Cy7, 6-FAM, dansyl chloride, fluorescein, HEX, 6-JOE, NBD (7-nitrobenzo-2-oxo-1,3-diazole), Oregon Green 488, Oregon Green 500, Oregon Green 514, Pacific Blue, phthalic acid, terephthalic acid, isophthalic acid, Cresol Fast Violet, Cresol Blue Violet, Brilliant Cresyl Blue, p-aminobenzoic acid, erythrosine, phthalocyanine, azomethine, cyanine, xanthine, succinyl fluorescein, rare earth metal cryptate, europium tribipyridyl diamine, europium cryptate or chelate, diamine, biscyanine, La Jolla blue dye, allophycocyanin, allococyanin B, phycocyanin C, phycocyanin R, thiamine, phycoerythrin, phycoerythrin R, REG, rhodamine green, rhodamine isothiocyanate, rhodamine red, ROX, TAMRA, TET, tetramethylrhodamine isothiol (TRIT), tetramethylrhodamine, and Texas Red.

In one or more embodiments, the radioisotope includes any one of ¹¹⁰In, ¹¹¹In, ¹⁷⁷Lu, ¹⁸F, ⁵²Fe, ⁶²Cu, ⁶⁴Cu, ⁶⁷Cu, ⁶⁷Ga, ⁶⁸Ga, ⁸⁶Y, ⁹⁰Y, ⁸⁹Zr, ⁹⁴mTc, ⁹⁴Tc, ⁹⁹mTc, ¹²⁰I, ¹²³I, ¹²⁴I, ¹²⁵I, ¹³¹I, ¹⁵⁴⁻¹⁵⁸Gd, ³²P, ¹¹C, ¹³N, ¹⁵O, ¹⁸⁶Re, ¹⁸⁸Re, ⁵¹Mn, ⁵²mMn, ⁵⁵Co, ⁷²As, ⁷⁵Br, ⁷⁶Br, ⁸²mRb, and ⁸³Sr.

In one or more embodiments, the enzyme includes any one of horseradish peroxidase, alkaline phosphatase, and glucose oxidase.

In one or more embodiments, the fluorescent microsphere is polystyrene fluorescent microsphere, coated with rare earth fluorescent ion europium inside.

In an aspect of the present disclosure, the present disclosure further relates to a kit including the binding protein as described above.

In one or more embodiments, the troponin I antigen is cardiac troponin I antigen.

The present disclosure further relates to a use of the binding protein as described above in a diagnosis of a cardiac troponin I-related disease.

The term "cardiac troponin I-related disease" as used herein refers to a disease that uses cardiac troponin I, including its protein or encoding nucleic acid, as a marker. In particular, in one or more embodiments of the present disclosure, the cardiac troponin I-related disease may refer to a disease characterized by an increase in a cardiac troponin I level in blood. In one or more embodiments of the present disclosure, the cardiac troponin I-related disease may refer to a disease characterized by a decrease in a cardiac troponin I level in cardiac muscle tissue or myocardial cells.

The present disclosure further relates to a method for diagnosing a cardiac troponin I-related disease. The method includes:
A) under conditions allowing a binding reaction to occur, contacting a sample from a subject with the binding protein as described in the present disclosure, to perform the binding reaction; and
B) detecting an immune complex produced in the binding reaction,
   in which a presence of the immune complex indicates a presence of the cardiac troponin I-related disease.

In one or more embodiments, the method is based on fluorescence immunoassay, chemiluminescence, colloidal gold immunoassay, radioimmunoassay, and/or enzyme-linked immunoassay.

In one or more embodiments, the sample is selected from at least one of whole blood, peripheral blood, serum, plasma, or myocardial tissue.

In one or more embodiments, the subject is a mammal, preferably a primate, and more preferably a human.

In one or more embodiments, the cardiac troponin I-related disease is a cardiovascular disease.

In one or more embodiments, the cardiac troponin I-related disease is selected from the group consisting of acute myocardial infarction, acute coronary syndrome, pulmonary infarction, unstable angina, myocardial injury, and combinations thereof.

The embodiments of the present disclosure are described in detail below in conjunction with examples, but those skilled in the art can understand that the following examples are merely for illustrating the present disclosure and shall not be considered as limitations of the scope of the present disclosure. The conventional conditions or the conditions recommended by the manufacturer shall be used where the specific conditions are not indicated in the examples. Reagents or instruments all conventional products that are commercially available unless the manufacturer is indicated.

### Example 1

This example provides an exemplary method for preparing a recombinant antibody against human cardiac troponin I.

### S10, constructing an expression plasmid:

In the present example, the restriction enzyme and Prime Star DNA polymerase were purchased from Takara Biomedical Technology Co., Ltd.;
MagExtractor-RNA extraction kit was purchased from TOYOBO Co., Ltd.; BD
SMART™ RACE cDNA Amplification Kit was purchased from Takara Biomedical Technology Co., Ltd.;
pMD-18T vector was purchased from Takara Biomedical Technology Co., Ltd.;
Plasmid extraction kit was purchased from Tiangen Biotech Co., Ltd.;
Primer synthesis and gene sequencing were performed by Invitrogen;

### S11, primer design and synthesis:

Amplification of heavy and light chain 5'RACE upstream primers:
Amplification of heavy and light chain 5'RACE primers:
SMARTER II A oligonucleotide:
5'>AAGCAGTGGTATCAACGCAGAGTACXXXXX <3';
5'-RACE CDS primer (5'-CDS):
5'>(T)₂₅VN<3' (N =A, C, G, or T; V = A, G, or C);
Universal Primer A Mixture (UPM):
5'>CTAATACGACTCACTATAGGGCAAGCAGTGGTATCAACGCAGAGT<3'
Nested Universal Primer A (NUP):
5'>AAGCAGTGGTATCAACGCAGAGT<3'
Amplification of downstream primer of light chain gene:
mkR:
5'>TTTTCCTTTTGAATTCCTAACACTCATTCCTGTTGAAGC<3'.
Amplification of downstream primer of heavy chain gene:
mHR:
5'>TTTTCCTTTTGAATTCTCATTTACCAGGAGAGTGGGAGA<3'.

### S12, gene cloning and sequencing of antibody variable region:

RNA was extracted from hybridoma cell line secreting Anti-cTnI 8C4 monoclonal antibody, and a first strand cDNA was synthesized by using SMARTER™ RACE cDNA Amplification Kit, SMARTER II A oligonucleotide in the kit, and 5'-CDS primers; and the obtained first strand cDNA product was used as a template for PCR amplification. Light chain genes were amplified with the universal primer A mixture (UPM), the nested universal primer A (NUP), and mkR primer; and heavy chain genes were amplified with the universal primer A mixture (UPM), the nested universal primer A (NUP), and mHR primer. Among them, the primer pair of light chain amplified a target band of about 0.8KB, and the primer pair of heavy chain amplified a target band of about 1.4KB. The product was purified and recovered by agarose gel electrophoresis, subjected to an A-tailing reaction with rTaq DNA polymerase, and then inserted into the pMD-18T vector, which was transformed into DH5α competent cells. After the growth of the bacteria, 4 clones of each of the heavy and light chain genes were taken and sent to Invitrogen for sequencing.

### S13, sequence analysis of the variable region genes of CTNI 8C4 antibody:

The gene sequence obtained by the above sequencing was analyzed in the IMGT antibody database, and analysis was performed using VNTI11.5 software to confirm that the genes amplified with the heavy chain and light chain primer pair were correct, among which, in the gene fragments amplified by light chain, the VL gene sequence was 342bp, belonging to the VkII gene family, with a 57bp leader peptide sequence in front; and in the gene fragments amplified by the heavy chain primer pair, the VH gene sequence was 357bp, belonging to the VH1 gene family, with 57bp leader peptide sequence in front.

### S 14, construction of recombinant antibody expression plasmid:

pcDNA™ 3.4 TOPO® vector was the constructed recombinant antibody eukaryotic expression vector. This expression vector had been introduced into polyclonal restriction sites such as HindIII, BamHI, EcoRI, and named as pcDNA3.4A expression vector, which is referred to as 3.4A expression vector below; according to the above pMD-18T antibody variable gene sequencing results, the VL gene-and VH gene-specific primers of the CTNI 8C4 antibody were designed, with HindIII, EcoRI restriction sites and protective bases at both ends, the primers are as follows:
C8C4-HF:
   5'>CAGCAAGCTTGCCGCCACCATGGAATGCAGCTGTGTCATGCTCTTCTTC<3';
C8C4-HR: 5'>CATCGAATTCTTATCATTTACCAGGAGAGTGGGAGA<3';
C8C4-LF:
   5'>CATCAAGCTTGCCGCCACCATGAAGTTGCCTGTTAGGCTGTTGG<3';
C8C4-LR: 5'>CAGCGAATTCTTACTAACACTCATTCCTGTTGAAGC<3';
   0.75KB light chain gene fragment and 1.42kb heavy chain gene fragment were amplified by PCR amplification method. The heavy and light chain gene fragments were double-enzyme digested with HindIII/EcoRI, and the 3.4A vector was double-enzyme digested with HindIII/EcoRI. After the fragment and the vector were purified and recovered, the heavy chain and light chain genes were ligated into the 3.4A expression vectors, respectively, so as to obtain recombinant expression plasmids for heavy and light chains, respectively.

The plasmid was diluted to 400 ng/ml with ultrapure water, and in a centrifuge tube, CHO cells were adjusted to 1.43×10⁷ cells/mL. 100 µl of the plasmid and 700 µl of the cells were mixed, transferred to an electro-revolving cup for electric revolving, then transferred to 10 ml of medium containing CD CHO AGT, and cultured in a shaker at 37°C (8% CO₂, shaking amplitude of 150); sample was taken to detect the cell viability every day, and when the cell viability was lower than 50%, the cell culture supernatant was centrifuged.

An affinity purification was performed using a protein A affinity chromatography column. 4 µg of the purified antibody was subjected to reduced SDS-PAGE, and two bands were shown after the reduced SDS-PAGE, one of which was a 28KD light chain (having sequence set forth as SEQ ID NO: 11), and the other one of which was a 50KD heavy chain (having sequence set forth as SEQ ID NO: 12).

### Example 2

The antibody obtained in Example 1 (having light and heavy chains whose sequences are set forth as SEQ ID NO: 11 and SEQ ID NO: 12) was analyzed, and the complementary determining region (WT) of the heavy chain:
CDR-VH1 is G-F-T-Y (X1)-S-N-Y-A-M-S;
CDR-VH2 is I-S-T (X1)-G-G-S-Y-S-Y (X2)-Y-P-D-S-V-K;
CDR-VH3 is A-R-K (X1)-D-N-Y-Y (X2)-G-S-T (X3)-F-M-D-Y;
the complementary determining region of the light chain:
CDR-VL1 is R-A-S-Q-D (X1)-I-T-Q (X2)-Y-L-N;
CDR-VL2 is I-Y-Y-S (X1)-S-R-I (X2)-H-S-G-V-S;
CDR-VL3 is Q-Q-G-N (X1)-T-I (X2)-P-L-T;
in which, X1, X2, X3 are mutation sites.

**[Table 1] Mutation sites related to antibody activity**

| Site | CDR-VH2 X1 | CDR-VH3 X1 | CDR-VL1 X2 | CDR-VL2 X1 |
|---|---|---|---|---|
| WT | T | K | Q | S |
| Mutation 1 | S | R | N | T |
| Mutation 2 | T | R | Q | T |
| Mutation 3 | S | K | N | S |
| Mutation 4 | S | K | Q | S |
| Mutation 5 | T | R | Q | S |

For obtaining antibodies with better activity, Applicant has made the above mutations on the CDR sites in WT.

CTNI quality control recombinant antigen was diluted to 1 µg/ml with a coating solution and coated a microplate, 100 µL per well, overnight at 4°C; on the next day, the microplate was washed with a washing solution twice and patted dry; a blocking solution (20% of BSA + 80% of PBS) was added, 120 µL per well, 37°C, 1h, patted dry; the diluted cTnI monoclonal antibodies were added, 100 µL/well, 37°C, 30 min; the microplate was washed with a washing solution 5 times, patted dry; goat anti-mouse IgG-HRP, 100 µL per well, 37°C, 30 min; the microplate was washed with a washing solution 5 times, patted dry; a color-developing solution A (50 µL/well) was added, a color-developing solution B (50 µL/well) was added, 10 min; a stop solution was added, 50 µL/well; OD values were read on microplate reader at 450 nm (reference, 630 nm).

**[Table 2] Analysis data of antibody activity**

| Activity Identification | | | | | | |
|---|---|---|---|---|---|---|
| Antibody concentration ng/ml | WT | Mutation 1 | Mutation 2 | Mutation 3 | Mutation 4 | Mutation 5 |
| 12.346 | 1.183 | 2.015 | 1.946 | 1.782 | 1.210 | 1.324 |
| 4.115 | 0.435 | 1.596 | 1.443 | 1.043 | 0.456 | 0.569 |
| 1.372 | 0.194 | 0.785 | 0.728 | 0.479 | 0.208 | 0.214 |
| 0.457 | 0.086 | 0.307 | 0.248 | 0.172 | 0.098 | 0.098 |
| 0.152 | 0.053 | 0.129 | 0.072 | 0.068 | 0.069 | 0.072 |
| 0.051 | 0.040 | 0.088 | 0.058 | 0.049 | 0.061 | 0.054 |
| 0 | 0.031 | 0.039 | 0 | 0.011 | 0.029 | 0.030 |

It can be seen from Table 2 that, Mutation 1 has the best effect on activity, and thus Mutation 1 was used as a framework sequence to screen mutation sites with better potency (ensuring that the activity of the antibody obtained by screening is similar to Mutation 1, antibody activity ± 10%). Some of the results are shown as follows.

**[Table 3] Mutation sites related to antibody affinity**

| Site | CDR-VH1 X1 | CDR-VH2 X2 | CDR-VH3 X2/X3 | CDR-VL1 X1 | CDR-VL2 X2 | CDR-VL3 X1/X2 |
|---|---|---|---|---|---|---|
| Mutation 1 | Y | Y | Y/T | D | I | N/I |
| Mutation 1-1 | F | F | Y/S | E | L | H/L |
| Mutation 1-2 | Y | F | P/T | E | I | N/I |
| Mutation 1-3 | F | Y | P/S | D | L | H/L |
| Mutation 1-4 | Y | Y | F/T | E | L | N/L |
| Mutation 1-5 | F | F | F/S | E | I | H/L |
| Mutation 1-6 | Y | F | Y/S | D | L | N/I |
| Mutation 1-7 | F | Y | F/T | E | I | H/I |
| Mutation 1-8 | Y | Y | F/S | E | I | H/I |
| Mutation 1-9 | F | F | Y/T | D | L | N/L |
| Mutation 1-10 | Y | F | Y/S | D | I | H/L |
| Mutation 1-11 | F | Y | Y/T | D | L | N/I |
| Mutation 1-12 | Y | Y | P/T | E | L | H/L |
| Mutation 1-13 | F | F | P/S | E | I | N/I |
| Mutation 1-14 | Y | F | F/T | D | L | H/I |
| Mutation 1-15 | F | Y | F/S | E | I | N/L |
| Mutation 1-16 | Y | Y | Y/T | D | I | N/I |
| Mutation 1-17 | F | F | Y/S | D | L | H/I |
| Mutation 1-18 | Y | F | P/S | E | I | N/L |
| Mutation 1-19 | F | Y | F/T | E | L | H/L |
| Mutation 1-20 | Y | Y | F/S | D | L | N/L |
| Mutation 1-21 | F | F | Y/T | D | I | H/L |
| Mutation 1-22 | Y | F | Y/S | D | I | H/I |
| Mutation 1-23 | F | Y | P/T | E | I | H/I |
| Mutation 1-24 | Y | Y | F/T | E | I | H/I |
| Mutation 1-25 | F | F | F/S | D | L | N/L |
| Mutation 1-26 | Y | F | Y/T | E | I | H/L |
| Mutation 1-27 | F | Y | Y/S | D | L | N/I |
| Mutation 1-28 | Y | Y | P/T | D | L | H/L |
| Mutation 1-29 | F | F | F/S | E | I | N/I |
| Mutation 1-30 | Y | F | F/S | E | L | H/I |
| Mutation 1-31 | F | Y | Y/T | D | I | N/L |
| Mutation 1-32 | Y | Y | Y/S | D | I | N/I |
| Mutation 1-33 | F | F | P/T | D | L | H/I |
| Mutation 1-34 | Y | F | P/S | D | I | N/I |
| Mutation 1-35 | F | Y | F/T | D | L | H/L |
| Mutation 1-36 | Y | Y | Y/T | E | I | N/L |
| Mutation 1-37 | F | F | Y/S | D | L | H/L |
| Mutation 1-38 | Y | F | P/T | D | L | N/I |
| Mutation 1-39 | F | Y | P/S | D | I | H/I |
| Mutation 1-40 | Y | Y | F/T | E | I | N/L |
| Mutation 1-41 | F | F | F/S | E | L | N/L |
| Mutation 1-42 | Y | F | Y/S | E | I | H/I |
| Mutation 1-43 | F | Y | P/T | D | L | N/I |
| Mutation 1-44 | Y | Y | P/S | D | L | H/L |
| Mutation 1-45 | F | F | F/T | E | I | N/I |
| Mutation 1-46 | Y | F | Y/T | E | L | H/L |
| Mutation 1-47 | F | Y | P/S | D | I | N/L |
| Mutation 1-48 | Y | Y | P/T | E | I | N/I |

### Affinity analysis

With an AMC sensor, the purified antibody was diluted to 10 µg/ml with PBST, and CTNI quality control recombinant protein (antigen produced by the company) was gradient diluted with PBST to 769.2 nmol/ml, 384.6 nmol/ml, 192.3 nmol/ml, 96.2 nmol/ml, 48.1 nmol/ml, 24 nmol/ml, 12 nmol/ml, and 0 nmol/ml.

Operating procedure: equilibrating for 60s in Buffer 1 (PBST), solidifying the antibody for 300s in the antibody solution, incubating for 180s in Buffer 2 (PBST), binding for 420s in the antigen solution, dissociating for 1200s in Buffer 2, and using 10mM of GLY solution (pH 1.69) and Buffer 3 to perform sensor regeneration, and outputting data. (K_{D} represents an equilibrium dissociation constant, which is a measure for affinity; kon represents a binding rate; and kdis represents a dissociation rate).

**[Table 4] Analysis data of affinity**

| Site | K_{D}(M) | K_{ON} (1/Ms) | kdis (1/s) |
|---|---|---|---|
| Mutation 1 | 7.04E-09 | 8.28E+04 | 5.83E-04 |
| Mutation 1-1 | 3.47E-09 | 3.58E+04 | 1.24E-04 |
| Mutation 1-2 | 2.43E-09 | 5.32E+04 | 1.29E-04 |
| Mutation 1-3 | 4.58E-09 | 1.25E+04 | 5.73E-05 |
| Mutation 1-4 | 7.10E-09 | 7.46E+04 | 5.30E-04 |
| Mutation 1-5 | 8.30E-10 | 9.72E+05 | 8.07E-04 |
| Mutation 1-6 | 9.81E-09 | 2.80E+04 | 2.75E-04 |
| Mutation 1-7 | 4.71E-09 | 3.94E+04 | 1.86E-04 |
| Mutation 1-8 | 6.81E-09 | 9.80E+04 | 6.67E-04 |
| Mutation 1-9 | 8.60E-09 | 2.14E+04 | 1.84E-04 |
| Mutation 1-10 | 5.05E-09 | 6.85E+04 | 3.46E-04 |
| Mutation 1-11 | 4.40E-09 | 1.08E+04 | 4.75E-05 |
| Mutation 1-12 | 1.52E-09 | 8.86E+04 | 1.35E-04 |
| Mutation 1-13 | 4.12E-09 | 8.60E+04 | 3.54E-04 |
| Mutation 1-14 | 7.76E-09 | 6.42E+04 | 4.98E-04 |
| Mutation 1-15 | 3.80E-09 | 3.81E+04 | 1.45E-04 |
| Mutation 1-16 | 9.72E-10 | 8.57E+05 | 8.33E-04 |
| Mutation 1-17 | 9.22E-09 | 3.64E+04 | 3.36E-04 |
| Mutation 1-18 | 4.40E-09 | 5.75E+04 | 2.53E-04 |
| Mutation 1-19 | 8.19E-09 | 9.43E+04 | 7.72E-04 |
| Mutation 1-20 | 2.87E-09 | 5.98E+04 | 1.72E-04 |
| Mutation 1-21 | 9.55E-09 | 6.62E+04 | 6.32E-04 |
| Mutation 1-22 | 9.07E-09 | 3.44E+04 | 3.12E-04 |
| Mutation 1-23 | 4.31E-09 | 7.79E+04 | 3.36E-04 |
| Mutation 1-24 | 5.37E-09 | 4.61E+04 | 2.48E-04 |
| Mutation 1-25 | 5.36E-09 | 6.79E+04 | 3.64E-04 |
| Mutation 1-26 | 1.38E-09 | 2.52E+04 | 3.48E-05 |
| Mutation 1-27 | 6.30E-09 | 7.96E+04 | 5.01E-04 |
| Mutation 1-28 | 3.68E-09 | 1.17E+04 | 4.31E-05 |
| Mutation 1-29 | 7.59E-09 | 2.60E+04 | 1.97E-04 |
| Mutation 1-30 | 5.76E-09 | 2.83E+04 | 1.63E-04 |
| Mutation 1-31 | 8.73E-09 | 8.52E+04 | 7.44E-04 |
| Mutation 1-32 | 8.38E-09 | 3.17E+04 | 2.66E-04 |
| Mutation 1-33 | 9.79E-10 | 5.32E+05 | 5.21E-04 |
| Mutation 1-34 | 8.44E-09 | 3.02E+04 | 2.55E-04 |
| Mutation 1-35 | 5.25E-09 | 3.07E+04 | 1.61E-04 |
| Mutation 1-36 | 9.27E-09 | 8.35E+04 | 7.74E-04 |
| Mutation 1-37 | 4.20E-09 | 7.10E+04 | 2.99E-04 |
| Mutation 1-38 | 7.74E-09 | 7.37E+04 | 5.70E-04 |
| Mutation 1-39 | 6.25E-09 | 8.14E+04 | 5.09E-04 |
| Mutation 1-40 | 3.12E-09 | 8.39E+04 | 2.62E-04 |
| Mutation 1-41 | 9.24E-09 | 8.38E+04 | 7.74E-04 |
| Mutation 1-42 | 3.09E-09 | 3.52E+04 | 1.09E-04 |
| Mutation 1-43 | 5.37E-09 | 4.61E+04 | 2.48E-04 |
| Mutation 1-44 | 3.53E-09 | 4.85E+04 | 1.71E-04 |
| Mutation 1-45 | 9.02E-09 | 2.11E+04 | 1.90E-04 |
| Mutation 1-46 | 4.93E-09 | 5.56E+04 | 2.74E-04 |
| Mutation 1-47 | 2.87E-09 | 3.30E+04 | 9.47E-05 |
| Mutation 1-48 | 7.04E-09 | 8.28E+04 | 5.83E-04 |

As can be seen from Table 4, the mutation sites listed in Table 3 have little effect on the affinity of the antibodies.

In order to verify the above results, the above experiments were repeated using WT as the framework sequence to verify the affinity of the mutation site. Some of the results are shown as follows.

**[Table 5] Mutations using WT as framework**

| Site | CDR-VH1 X1 | CDR-VH2 X2 | CDR-VH3 X2/X3 | CDR-VL1 X1 | CDR-VL2 X2 | CDR-VL3 X1/X2 |
|---|---|---|---|---|---|---|
| WT | Y | Y | Y/T | D | I | N/I |
| WT 1-7 | F | Y | P/T | E | I | H/I |
| WT 1-16 | Y | Y | Y/T | D | I | N/I |
| WT 1-25 | F | F | F/S | D | L | N/L |
| WT 1-35 | F | Y | F/T | E | L | H/L |

**[Table 6] Analysis data of affinity**

| Site | K_{D} (M) | K_{ON} (1/Ms) | Kdis (1/s) |
|---|---|---|---|
| WT | 2.16E-08 | 7.83E+03 | 1.69E-04 |
| WT 1-1 | 3.33E-08 | 3.84E+03 | 1.28E-04 |
| WT 1-5 | 8.35E-09 | 8.90E+04 | 7.43E-04 |
| WT 1-10 | 4.89E-09 | 2.65E+04 | 1.30E-04 |
| WT 1-20 | 9.39E-09 | 2.41E+04 | 2.26E-04 |

By analyzing Table 5 and Table 6, on the premise of ensuring antibody activity, the above mutation sites also have a certain affinity.

It should be noted that the above embodiments are only used to illustrate the technical solutions of the present disclosure, rather than limiting them. Although the present disclosure has been described in detail with reference to the foregoing embodiments, those skilled in the art should understand that they can modify the technical solutions described in the foregoing embodiments or replace some or all of the technical features equivalently; and these modifications or replacements do not deviate the essence of the corresponding technical solutions from the scope of the technical solutions of the embodiments of the present disclosure.

### Industrial Applicability

In the present disclosure, the isolated binding protein including an antigen-binding domain that binds to cardiac troponin I, includes specific heavy chain CDRs and light chain CDRs. The binding protein can specifically recognize and bind to cardiac troponin I protein, and has relatively high sensitivity and specificity. Particularly, the binding protein has high affinity to human cTnI protein. Thus, the detection and diagnosis of the cardiac troponin I-related diseases can be achieved.

## Claims

1. An isolated binding protein comprising an antigen-binding domain, wherein the antigen-binding domain comprises at least one complementary determining region selected from the following amino acid sequences, or the antigen-binding domain has at least 80% sequence identity with the complementary determining regions of the following amino acid sequences and has an affinity for cardiac troponin I of K_{D} ≤ 3.33×10⁻⁸ mo/L:
a complementary determining region CDR-VH1 of G-F-T-X1-S-N-Y-A-M-S, where X1 is Y or F;
a complementary determining region CDR-VH2 of I-S-X1-G-G-S-Y-S-X2-Y-P-D-S-V-K, where X1 is S or T, and X2 is Y or F;
a complementary determining region CDR-VH3 of A-R-X1-D-N-Y-X2-G-S-X3-F-M-D-Y, where X1 is R or K, X2 is Y, P or F, and X3 is T or S;
a complementary determining region CDR-VL1 of R-A-S-Q-X1-I-T-X2-Y-L-N, where X1 is D or E, and X2 is N or Q;
a complementary determining region CDR-VL2 of I-Y-Y-X1-S-R-X2-H-S-G-V-S, where X1 is T or S, and X2 is I or L; and
a complementary determining region CDR-VL3 of Q-Q-G-X1-T-X2-P-L-T, where X1 is N or H, and X2 is I or L,
preferably,
in the complementary determining region CDR-VH2, X1 is S;
in the complementary determining region CDR-VH3, X1 is R;
in the complementary determining region CDR-VL1, X2 is N; and
in the complementary determining region CDR-VL2, X1 is T, preferably, in the complementary determining region CDR-VH1, X1 is Y;
preferably, in the complementary determining region CDR-VH1, X1 is F;
preferably, in the complementary determining region CDR-VH2, X2 is Y;
preferably, in the complementary determining region CDR-VH2, X2 is F;
preferably, in the complementary determining region CDR-VH3, X2 is Y, and X3 is T;
preferably, in the complementary determining region CDR-VH3, X2 is P, and X3 is T;
preferably, in the complementary determining region CDR-VH3, X2 is F, and X3 is T;
preferably, in the complementary determining region CDR-VH3, X2 is Y, and X3 is S;
preferably, in the complementary determining region CDR-VH3, X2 is P, and X3 is S;
preferably, in the complementary determining region CDR-VH3, X2 is F, and X3 is S;
preferably, in the complementary determining region CDR-VL1, X1 is D;
preferably, in the complementary determining region CDR-VL1, X1 is E;
preferably, in the complementary determining region CDR-VL2, X2 is I;
preferably, in the complementary determining region CDR-VL2, X2 is L;
preferably, in the complementary determining region CDR-VL3, X1 is N, and X2 is I;
preferably, in the complementary determining region CDR-VL3, X1 is H, and X2 is I;
preferably, in the complementary determining region CDR-VL3, X1 is N, and X2 is L;
preferably, in the complementary determining region CDR-VL3, X1 is H, and X2 is L; and
preferably, a mutation site of each of the complementary determining regions is selected from any one of the following mutation combinations:
| Site | CDR-VH1 X1 | CDR-VH2 X2 | CDR-VH3 X2/X3 | CDR-VL1 X1 | CDR-VL2 X2 | CDR-VL3 X1/X2 |
|---|---|---|---|---|---|---|
| Mutation combination 1 | Y | Y | Y/T | D | I | N/I |
| Mutation combination 2 | F | F | Y/S | E | L | H/L |
| Mutation combination 3 | Y | F | P/T | E | I | N/I |
| Mutation combination 4 | F | Y | P/S | D | L | H/L |
| Mutation combination 5 | Y | Y | F/T | E | L | N/L |
| Mutation combination 6 | F | F | F/S | E | I | H/L |
| Mutation combination 7 | Y | F | Y/S | D | L | N/I |
| Mutation combination 8 | F | Y | F/T | E | I | H/I |
| Mutation combination 9 | Y | Y | F/S | E | I | H/I |
| Mutation combination 10 | F | F | Y/T | D | L | N/L |
| Mutation combination 11 | Y | F | Y/S | D | I | H/L |
| Mutation combination 12 | F | Y | Y/T | D | L | N/I |
| Mutation combination 13 | Y | Y | P/T | E | L | H/L |
| Mutation combination 14 | F | F | P/S | E | I | N/I |
| Mutation combination 15 | Y | F | F/T | D | L | H/I |
| Mutation combination 16 | F | Y | F/S | E | I | N/L |
| Mutation combination 17 | Y | Y | Y/T | D | I | N/I |
| Mutation combination 18 | F | F | Y/S | D | L | H/I |
| Mutation combination 19 | Y | F | P/S | E | I | N/L |
| Mutation combination 20 | F | Y | F/T | E | L | H/L |
| Mutation combination 21 | Y | Y | F/S | D | L | N/L |
| Mutation combination 22 | F | F | Y/T | D | I | H/L |
| Mutation combination 23 | Y | F | Y/S | D | I | H/I |
| Mutation combination 24 | F | Y | P/T | E | I | H/I |
| Mutation combination 25 | Y | Y | F/T | E | I | H/I |
| Mutation combination 26 | F | F | F/S | D | L | N/L |
| Mutation combination 27 | Y | F | Y/T | E | I | H/L |
| Mutation combination 28 | F | Y | Y/S | D | L | N/I |
| Mutation combination 29 | Y | Y | P/T | D | L | H/L |
| Mutation combination 30 | F | F | F/S | E | I | N/I |
| Mutation combination 31 | Y | F | F/S | E | L | H/I |
| Mutation combination 32 | F | Y | Y/T | D | I | N/L |
| Mutation combination 33 | Y | Y | Y/S | D | I | N/I |
| Mutation combination 34 | F | F | P/T | D | L | H/I |
| Mutation combination 35 | Y | F | P/S | D | I | N/I |
| Mutation combination 36 | F | Y | F/T | D | L | H/L |
| Mutation combination 37 | Y | Y | Y/T | E | I | N/L |
| Mutation combination 38 | F | F | Y/S | D | L | H/L |
| Mutation combination 39 | Y | F | P/T | D | L | N/I |
| Mutation combination 40 | F | Y | P/S | D | I | H/I |
| Mutation combination 41 | Y | Y | F/T | E | I | N/L |
| Mutation combination 42 | F | F | F/S | E | L | N/L |
| Mutation combination 43 | Y | F | Y/S | E | I | H/I |
| Mutation combination 44 | F | Y | P/T | D | L | N/I |
| Mutation combination 45 | Y | Y | P/S | D | L | H/L |
| Mutation combination 46 | F | F | F/T | E | I | N/I |
| Mutation combination 47 | Y | F | Y/T | E | L | H/L |
| Mutation combination 48 | F | Y | P/S | D | I | N/L |
| Mutation combination 49 | Y | Y | P/T | E | I | N/I |

2. The isolated binding protein comprising the antigen-binding domain according to claim 1, wherein the binding protein comprises at least 3 CDRs; or the binding protein comprises at least 6 CDRs;
preferably, the binding protein is one of nanobody, F(ab')₂, Fab', Fab, Fv, scFv, a bispecific antibody, and a minimum recognition unit of an antibody; and
preferably, the binding protein comprises light chain framework regions FR-L1, FR-L2, FR-L3, and FR-L4 that have sequences set forth as SEQ ID NO: 1 to SEQ ID NO: 4, respectively, and/or heavy chain framework regions FR-H1, FR-H2, FR-H3 and FR-H4 that have sequences set forth as SEQ ID NO: 5 to SEQ ID NO: 8, respectively.

3. The isolated binding protein comprising the antigen-binding domain according to claim 2, wherein the binding protein further comprises an antibody constant region sequence;
preferably, the constant region sequence is a sequence of constant region selected from any one of IgG1, IgG2, IgG3, IgG4, IgA, IgM, IgE, and IgD;
preferably, the constant region is derived from a species of cattle, horse, dairy cow, pig, sheep, goat, rat, mouse, dog, cat, rabbit, camel, donkey, deer, marten, chicken, duck, goose, turkey, cockfight, or human;
preferably, the constant region is derived from a mouse;
a light chain constant region sequence is set forth as SEQ ID NO: 9; and
a heavy chain constant region sequence is set forth as SEQ ID NO: 10.

4. An isolated nucleic acid molecule, wherein the nucleic acid molecule is DNA or RNA that encodes the binding protein according to any one of claims 1 to 3.

5. A vector, comprising the nucleic acid molecule according to claim 4.

6. A host cell, transformed with the vector according to claim 5.

7. A method for producing the binding protein according to any one of claims 1 to 3, the method comprising the following steps:
culturing the host cell according to claim 6 in a medium under suitable culture conditions, and collecting the produced binding protein from the medium or from the cultured host cell.

8. Use of the binding protein according to any one of claims 1 to 3 in a preparation of a diagnostic agent or kit for diagnosing acute myocardial infarction, acute coronary syndrome, pulmonary infarction, unstable angina, or myocardial injury.

9. A method for detecting a troponin I antigen in a test sample, the method comprising:
a) under conditions allowing an antibody/antigen binding reaction to occur, contacting the troponin I antigen in the test sample with the binding protein according to any one of claims 1 to 3 to form an immune complex; and
b) detecting a presence of the immune complex, the presence of the immune complex indicating a presence of the troponin I antigen in the test sample,
preferably, in step a), the immune complex further comprising a second antibody, and the second antibody binding to the binding protein,
preferably, in step a), the immune complex further comprising a second antibody, and the second antibody binding to the troponin I antigen.

10. The method according to claim 9, wherein the troponin I antigen is cardiac troponin I antigen.

11. A kit, comprising the binding protein according to any one of claims 1 to 3.

12. Use of the binding protein according to any one of claims 1 to 3 in a diagnosis of a cardiac troponin I-related disease.

13. A method for diagnosing a cardiac troponin I-related disease, the method comprising:
A) under conditions allowing a binding reaction to occur, contacting a sample from a subject with the binding protein according to any one of claims 1 to 3, to perform the binding reaction; and
B) detecting an immune complex produced in the binding reaction,
wherein a presence of the immune complex indicates a presence of the cardiac troponin I-related disease.

14. The method according to claim 13, wherein the method is based on fluorescence immunoassay, chemiluminescence, colloidal gold immunoassay, radioimmunoassay, and/or enzyme-linked immunoassay.

15. The method according to claim 13 or 14, wherein the sample is selected from at least one of whole blood, peripheral blood, serum, plasma, or myocardial tissue.

16. The method according to any one of claims 13 to 15, wherein the subject is a mammal, preferably a primate, and more preferably a human.

17. The use according to claim 12 or the method according to any one of claims 13 to 16, wherein the cardiac troponin I-related disease is a cardiovascular disease.

18. The use according to claim 12 or the method according to any one of claims 13 to 16, wherein the cardiac troponin I-related disease is selected from the group consisting of acute myocardial infarction, acute coronary syndrome, pulmonary infarction, unstable angina, myocardial injury, and combinations thereof.


Sequence Listing

<110>  DONGGUAN PENGZHI BIOTECHNOLOGY CO., LTD.

<120>  RECOMBINANT ANTIBODY AGAINST HUMAN CARDIAC TROPONIN I

<130>  PI19035800

<150>  CN2018111803628
<151>  2018-10-10

<160>  12

<170>  PatentIn version 3.3

<210>  1
<211>  23
<212>  PRT
<213>  Mus musculus

<400>  1

Asp Ile Gln Met Thr Gln Thr Ser Ser Ser Leu Ser Ala Ser Leu Gly
1               5                   10                  15

Asp Arg Val Thr Ile Ser Cys
            20

<210>  2
<211>  13
<212>  PRT
<213>  Mus musculus

<400>  2

Trp Tyr Gln Gln Lys Pro Asp Gly Thr Ile Lys Leu Leu
1               5                   10

<210>  3
<211>  29
<212>  PRT
<213>  Mus musculus

<400>  3

Ser Arg Phe Ser Gly Ser Gly Ser Gly Thr Asp Tyr Ser Leu Thr Ile
1               5                   10                  15

Ser Asn Leu Glu Gln Glu Asp Ile Ala Thr Tyr Phe Cys
            20                  25

<210>  4
<211>  10

<212>  PRT
<213>  Mus musculus

<400>  4

Phe Asp Thr Gly Thr Lys Leu Glu Leu Lys
1               5                   10


<210>  5
<211>  25
<212>  PRT
<213>  Mus musculus

<400>  5

Glu Val Gln Met Val Glu Ser Gly Gly Gly Leu Leu Lys Pro Gly Gly
1               5                   10                  15


Ser Leu Lys Leu Ser Cys Ala Ala Ser
            20                  25


<210>  6
<211>  15
<212>  PRT
<213>  Mus musculus

<400>  6

Trp Val Arg Gln Thr Pro Glu Lys Arg Leu Glu Trp Val Ala Thr
1               5                   10                  15


<210>  7
<211>  31
<212>  PRT
<213>  Mus musculus

<400>  7

Gly Arg Phe Thr Ile Ser Arg Asp Asn Ala Lys Asn Thr Leu Tyr Leu
1               5                   10                  15


Gln Met Leu Ser Leu Arg Ser Glu Asp Thr Ala Met Tyr Tyr Cys
            20                  25                  30


<210>  8
<211>  11
<212>  PRT
<213>  Mus musculus

<400>  8

Trp Gly Gln Gly Ser Ser Val Thr Val Ser Ser

1               5                   10

<210>  9
<211>  107
<212>  PRT
<213>  Mus musculus

<400>  9

Arg Ala Asp Ala Ala Pro Thr Val Ser Ile Phe Pro Pro Ser Ser Glu
1               5                   10                  15

Gln Leu Thr Ser Gly Gly Ala Ser Val Val Cys Phe Leu Asn Asn Phe
            20                  25                  30

Tyr Pro Lys Asp Ile Asn Val Lys Trp Lys Ile Asp Gly Ser Glu Arg
        35                  40                  45

Gln Asn Gly Val Leu Asn Ser Trp Thr Asp Gln Asp Ser Lys Asp Ser
    50                  55                  60

Thr Tyr Ser Met Ser Ser Thr Leu Thr Leu Thr Lys Asp Glu Tyr Glu
65                  70                  75                  80

Arg His Asn Ser Tyr Thr Cys Glu Ala Thr His Lys Thr Ser Thr Ser
                85                  90                  95

Pro Ile Val Lys Ser Phe Asn Arg Asn Glu Cys
            100                 105

<210>  10
<211>  323
<212>  PRT
<213>  Mus musculus

<400>  10

Ala Lys Thr Thr Pro Pro Ser Val Tyr Pro Leu Ala Pro Gly Ser Ala
1               5                   10                  15

Ala Gln Thr Asn Ser Met Val Thr Leu Gly Cys Leu Val Lys Gly Tyr
            20                  25                  30

Phe Pro Glu Pro Val Thr Val Thr Trp Asn Ser Gly Ser Leu Ser Ser
        35                  40                  45

Gly Val His Thr Phe Pro Ala Val Leu Gln Ser Asp Leu Tyr Thr Leu

50                  55                  60

Ser Ser Ser Val Thr Val Pro Ser Ser Thr Trp Pro Ser Gln Thr Val
65                  70                  75                  80

Thr Cys Asn Val Ala His Pro Ala Ser Ser Thr Lys Val Asp Lys Lys
                85                  90                  95

Ile Val Pro Arg Asp Cys Gly Cys Lys Pro Cys Ile Cys Thr Val Pro
            100                 105                 110

Glu Val Ser Ser Val Phe Ile Phe Pro Pro Lys Pro Lys Asp Val Leu
        115                 120                 125

Thr Ile Thr Leu Thr Pro Lys Val Thr Cys Val Val Val Asp Ile Ser
    130                 135                 140

Lys Asp Asp Pro Glu Val Gln Phe Ser Trp Phe Val Asp Asp Val Glu
145                 150                 155                 160

Val His Thr Ala Gln Thr Lys Pro Arg Glu Glu Gln Phe Asn Ser Thr
                165                 170                 175

Phe Arg Ser Val Ser Glu Leu Pro Ile Met His Gln Asp Trp Leu Asn
            180                 185                 190

Gly Lys Glu Phe Lys Cys Arg Val Asn Ser Ala Ala Phe Pro Ala Pro
        195                 200                 205

Ile Glu Lys Thr Ile Ser Lys Thr Lys Gly Arg Pro Lys Ala Pro Gln
    210                 215                 220

Val Tyr Thr Ile Pro Pro Pro Lys Glu Gln Met Ala Lys Asp Lys Val
225                 230                 235                 240

Ser Leu Thr Cys Met Ile Thr Asn Phe Phe Pro Glu Asp Ile Thr Val
                245                 250                 255

Glu Trp Gln Trp Asn Gly Gln Pro Ala Glu Asn Tyr Lys Asn Thr Gln
            260                 265                 270

Pro Ile Met Asp Thr Asp Gly Ser Tyr Phe Val Tyr Ser Lys Leu Asn
        275                 280                 285

Val Gln Lys Ser Asn Trp Glu Ala Gly Asn Thr Phe Thr Cys Ser Val
    290                 295                 300

Leu His Glu Gly Leu His Asn His His Thr Glu Lys Ser Leu Ser His
305                 310                 315                 320

Ser Pro Gly

<210>  11
<211>  214
<212>  PRT
<213>  Artificial sequence

<220>
<223>  Light chain

<400>  11

Asp Ile Gln Met Thr Gln Thr Ser Ser Ser Leu Ser Ala Ser Leu Gly
1               5                   10                  15

Asp Arg Val Thr Ile Ser Cys Arg Ala Ser Gln Asp Ile Thr Gln Tyr
            20                  25                  30

Leu Asn Trp Tyr Gln Gln Lys Pro Asp Gly Thr Ile Lys Leu Leu Ile
        35                  40                  45

Tyr Tyr Ser Ser Arg Ile His Ser Gly Val Ser Ser Arg Phe Ser Gly
    50                  55                  60

Ser Gly Ser Gly Thr Asp Tyr Ser Leu Thr Ile Ser Asn Leu Glu Gln
65                  70                  75                  80

Glu Asp Ile Ala Thr Tyr Phe Cys Gln Gln Gly Asn Thr Ile Pro Leu
                85                  90                  95

Thr Phe Asp Thr Gly Thr Lys Leu Glu Leu Lys Arg Ala Asp Ala Ala
            100                 105                 110

Pro Thr Val Ser Ile Phe Pro Pro Ser Ser Glu Gln Leu Thr Ser Gly
        115                 120                 125

Gly Ala Ser Val Val Cys Phe Leu Asn Asn Phe Tyr Pro Lys Asp Ile
    130                 135                 140

Asn Val Lys Trp Lys Ile Asp Gly Ser Glu Arg Gln Asn Gly Val Leu
145                 150                 155                 160

Asn Ser Trp Thr Asp Gln Asp Ser Lys Asp Ser Thr Tyr Ser Met Ser
                165                 170                 175

Ser Thr Leu Thr Leu Thr Lys Asp Glu Tyr Glu Arg His Asn Ser Tyr
            180                 185                 190

Thr Cys Glu Ala Thr His Lys Thr Ser Thr Ser Pro Ile Val Lys Ser
        195                 200                 205

Phe Asn Arg Asn Glu Cys
    210

<210>  12
<211>  444
<212>  PRT
<213>  Artificial sequence

<220>
<223>  Heavy chain

<400>  12

Glu Val Gln Met Val Glu Ser Gly Gly Gly Leu Leu Lys Pro Gly Gly
1               5                   10                  15

Ser Leu Lys Leu Ser Cys Ala Ala Ser Gly Phe Thr Tyr Ser Asn Tyr
            20                  25                  30

Ala Met Ser Trp Val Arg Gln Thr Pro Glu Lys Arg Leu Glu Trp Val
        35                  40                  45

Ala Thr Ile Ser Thr Gly Gly Ser Tyr Ser Tyr Tyr Pro Asp Ser Val
    50                  55                  60

Lys Gly Arg Phe Thr Ile Ser Arg Asp Asn Ala Lys Asn Thr Leu Tyr
65                  70                  75                  80

Leu Gln Met Leu Ser Leu Arg Ser Glu Asp Thr Ala Met Tyr Tyr Cys
                85                  90                  95

Ala Arg Lys Asp Asn Tyr Tyr Gly Ser Thr Phe Met Asp Tyr Trp Gly
            100                 105                 110

Gln Gly Ser Ser Val Thr Val Ser Ser Ala Lys Thr Thr Pro Pro Ser
        115                 120                 125

Val Tyr Pro Leu Ala Pro Gly Ser Ala Ala Gln Thr Asn Ser Met Val
    130                 135                 140

Thr Leu Gly Cys Leu Val Lys Gly Tyr Phe Pro Glu Pro Val Thr Val
145                 150                 155                 160

Thr Trp Asn Ser Gly Ser Leu Ser Ser Gly Val His Thr Phe Pro Ala
                165                 170                 175

Val Leu Gln Ser Asp Leu Tyr Thr Leu Ser Ser Ser Val Thr Val Pro
            180                 185                 190

Ser Ser Thr Trp Pro Ser Gln Thr Val Thr Cys Asn Val Ala His Pro
        195                 200                 205

Ala Ser Ser Thr Lys Val Asp Lys Lys Ile Val Pro Arg Asp Cys Gly
    210                 215                 220

Cys Lys Pro Cys Ile Cys Thr Val Pro Glu Val Ser Ser Val Phe Ile
225                 230                 235                 240

Phe Pro Pro Lys Pro Lys Asp Val Leu Thr Ile Thr Leu Thr Pro Lys
                245                 250                 255

Val Thr Cys Val Val Val Asp Ile Ser Lys Asp Asp Pro Glu Val Gln
            260                 265                 270

Phe Ser Trp Phe Val Asp Asp Val Glu Val His Thr Ala Gln Thr Lys
        275                 280                 285

Pro Arg Glu Glu Gln Phe Asn Ser Thr Phe Arg Ser Val Ser Glu Leu
    290                 295                 300

Pro Ile Met His Gln Asp Trp Leu Asn Gly Lys Glu Phe Lys Cys Arg
305                 310                 315                 320

Val Asn Ser Ala Ala Phe Pro Ala Pro Ile Glu Lys Thr Ile Ser Lys
                325                 330                 335

Thr Lys Gly Arg Pro Lys Ala Pro Gln Val Tyr Thr Ile Pro Pro Pro

340                 345                 350

Lys Glu Gln Met Ala Lys Asp Lys Val Ser Leu Thr Cys Met Ile Thr
        355                 360                 365

Asn Phe Phe Pro Glu Asp Ile Thr Val Glu Trp Gln Trp Asn Gly Gln
    370                 375                 380

Pro Ala Glu Asn Tyr Lys Asn Thr Gln Pro Ile Met Asp Thr Asp Gly
385                 390                 395                 400

Ser Tyr Phe Val Tyr Ser Lys Leu Asn Val Gln Lys Ser Asn Trp Glu
                405                 410                 415

Ala Gly Asn Thr Phe Thr Cys Ser Val Leu His Glu Gly Leu His Asn
            420                 425                 430

His His Thr Glu Lys Ser Leu Ser His Ser Pro Gly
        435                 440